(19) Europäisches Patentamt
European Patent Office
Office européen des brevets

(11) Veröffentlichungsnummer: **0 153 601**
**B1**

(12) # EUROPÄISCHE PATENTSCHRIFT

(45) Veröffentlichungstag der Patentschrift: **01.04.87**

(51) Int. Cl.⁴: **C 07 D 239/42**, A 01 N 47/36

(21) Anmeldenummer: **85100969.6**

(22) Anmeldetag: **31.01.85**

(54) **2-Alkoxyaminosulfonylbenzolsulfonyl-harnstoff-Derivate.**

(30) Priorität: **10.02.84 JP 21839/84**

(43) Veröffentlichungstag der Anmeldung: **04.09.85 Patentblatt 85/36**

(45) Bekanntmachung des Hinweises auf die Patenterteilung: **01.04.87 Patentblatt 87/14**

(84) Benannte Vertragsstaaten: **AT BE CH DE FR GB IT LI NL**

(56) Entgegenhaltungen: **EP - A - 0 023 141**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Shiokawa, Kozo, 210-6, Shukugawara Tama-ku, Kawasaki-shi Kanagawa-ken (JP)**
Erfinder: **Goto, Toshio, 5-20-1-304, Seishin, Sagamihara-shi Kanagawa-ken (JP)**
Erfinder: **Kamochi, Atsumi, 2-24-10, Higashi-Toyoda, Hino-shi Tokyo (JP)**
Erfinder: **Moriya, Koichi, 39-15, Namiki-cho, Hachioji-shi Tokyo (JP)**
Erfinder: **Kohama, Shigeo, 39-15, Namiki-cho, Hachioji-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 153 601 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Alkoxyaminosulfonylbenzolsulfonyl-harnstoff-Derivate, Verfahren zu ihrer Herstellung sowie ihre Verwendung als Herbizide.

Insbesondere betrifft die vorliegende Erfindung 2-Alkoxyaminosulfonylbenzolsulfonyl-harnstoff-Derivate der nachstehenden allgemeinen Formel (I).

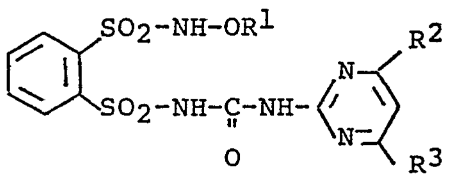

(I)

In der Formel bezeichnen $R^1$, $R^2$ und $R^3$ jeweils eine Alkyl-Gruppe mit 1 bis 4 Kohlenstoff-Atomen.

Die neuen Verbindungen der allgemeinen Formel (I) können mittels des folgenden Verfahrens hergestellt werden, auf die sich die vorliegende Erfindung ebenfalls erstreckt:

*Verfahren i)*

Das Verfahren zur Herstellung der 2-Alkoxyaminosulfonylbenzolsulfonyl-harnstoff-Derivate der allgemeinen Formel (I) umfasst die Umsetzung einer Verbindung der Formel

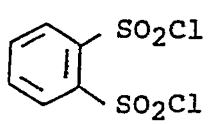

mit einer Verbindung der allgemeinen Formel

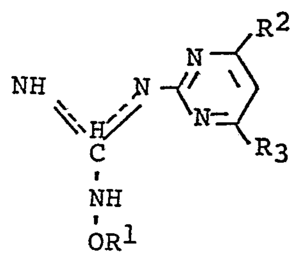

(II)

in der $R^1$, $R^2$ und $R^3$ die im Vorstehenden angegebene Bedeutung haben,
in Gegenwart einer Base, die anschliessende Umsetzung des Produkts mit einem Alkalimetallhydroxid der allgemeinen Formel

$$M-OH \qquad (III)$$

in der M ein Alkalimetall-Atom bezeichnet,
und weiterhin die Reaktion des Produkts mit einer anorganischen Säure.

Die Erfindung betrifft ausserdem Herbizide, die ein 2-Alkoxyaminosulfonylbenzolsulfonyl-harnstoff-Derivat der allgemeinen Formel (I) als Wirkstoff enthalten.

Die vor der Anmeldung der vorliegenden Erfindung bekannte EP-A-23141 stellt fest, dass Verbindungen der allgemeinen Formel

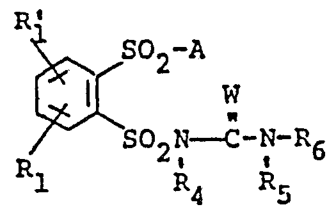

(Z)

herbizide Aktivität besitzen und offenbart eine Verbindung der folgenden Formel:

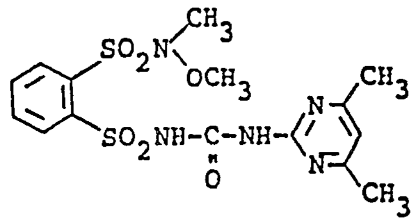

(Z-1)

Wenn jedoch A in der allgemeinen Formel (Z) $-NR_2R_3$ darstellt, schliessen die Definitionen für $R_2$ und $R_3$ in dem oben zitierten Dokument nicht eine Verbindung ein, die A = $-NH-OR^1$ entspricht, so wie dies in der vorliegenden Erfindung speziell angegeben ist. Die Verbindungen der Formel (I) gemäss der vorliegenden Erfindung können nicht mittels des in dem vorstehenden Dokument offenbarten Verfahrens hergestellt werden. Sie können jedoch mittels des oben beschriebenen Verfahrens gemäss der vorliegenden Erfindung hergestellt werden.

Seitens der Anmelderin wurden eingehende Untersuchungen mit dem Ziel der Schaffung neuer Verbindungen mit herbizider Wirkung durchgeführt. Im Zuge dieser Untersuchung wurden mit Erfolg die Verbindungen der allgemeinen Formel (I) synthetisiert, und es wurde gefunden, dass diese Verbindungen ausgezeichnete herbizide Aktivität besitzen. Auf diesen Befunden beruht die vorliegende Erfindung.

Nach bester Kenntnis der Anmelderin sind die Verbindungen der allgemeinen Formel (I) gemäss der vorliegenden Erfindung neue Verbindungen, die nicht in vor dem Einreichungszeitpunkt der vorliegenden Anmeldung erschienenen Veröffentlichungen beschrieben sind. Die Verbindungen der vorliegenden Erfindung sind gekennzeichnet durch die Tatsache, wie sie auch in der allgemeinen Formel (I) zum Ausdruck kommt, dass in ihrer chemischen Struktur N-Benzolsulfonyl-N'-(4,6-dialkylpyrimidin-2-yl)harnstoff als das Grundskelett vorliegt und eine Alkoxyaminosulfonyl-Gruppe in der 2-Stellung des Benzols substituiert ist. Die Verbindungen gemäss der vorliegenden Erfindung sind in ihrer chemischen Struktur den in dem oben zitierten Dokument ähnlich. Es wurde jedoch gefunden, dass die Verbindungen gemäss der vorliegenden Erfindung mit der oben bezeichneten charakteristischen chemischen Struktur nur mittels des Verfahrens i) gemäss der vorliegenden Erfindung hergestellt werden können, wohingegen sie nicht mittels des Verfahrens zur Herstellung der in dem oben zitierten Dokument spezifizierten Verbindung gewonnen werden können.

Biologisch besitzen die Verbindungen der vorliegenden Erfindung eine hervorragende, im Nutzpflanzenanbau auf höher gelegenen Feldern nutzbare selektive herbizide Aktivität dahingehend, dass sie eine starke herbizide Wirksamkeit gegenüber den betreffenden Acker-Unkräutern besitzen und keinerlei Phytotoxizität gegenüber den Nutzpflanzen auf höher gelegenen Feldern wie Sojabohnen und Winterweizen entfalten. Es wurde gefunden, dass die erwähnte Aktivität nur im Zusammenhang mit der durch die vorstehende allgemeine Formel dargestellten chemischen Struktur auftritt.

Die Verbindungen der vorliegenden Erfindung können beispielsweise leicht mittels des oben angegebenen Verfahrens i) hergestellt werden.

Ziel der vorliegenden Erfindung ist es demgemäss, die neuen 2-Alkoxyaminosulfonylbenzolsulfonyl-harnstoff-Derivate der allgemeinen Formel (I), ein Verfahren zur Herstellung derselben sowie deren Verwendung als Herbizide verfügbar zu machen.

Dieses sowie weitere Ziele der vorliegenden Erfindung werden aus der folgenden Beschreibung im einzelnen deutlich.

Die Verbindungen gemäss der vorliegenden Erfindung können mit Vorteil zur Unkrautbekämpfung eingesetzt werden, weil sie nur geringe Toxizität und eine gute Selektivität gegenüber Kulturpflanzen besitzen, oder, mit anderen Worten, in den üblichen Dosierungen keinerlei Phytotoxizität gegen Kulturpflanzen aufweisen. Die Herbizide gemäss der vorliegenden Erfindung zeigen eine hervorragende selektive Bekämpfungswirksamkeit insbesondere beim Einsatz gegen ein breites Spektrum von Ackerunkräutern als Mittel zur Bodenbehandlung vor dem Auflaufen oder als Mittel zur Laub- und Bodenbehandlung auf höher gelegenen Anbauflächen.

Die erfindungsgemässen Verbindungen der allgemeinen Formel (I) besitzen hohe Sicherheit und zeigen eine herausragende herbizide Wirksamkeit. Das herbizide Spektrum zeigt, dass sie starke herbizide Wirksamkeit entfalten, zum Beispiel gegen

Alopecurus aequalis Sobol. var. amurensis Ohwi,
Setaria glauca P. Beauv.,
Stellaria media Villars,
Echinochloa crus-galli P. Beauv.,
Digitaria adscendens Henr.,
Eleusine indica Gaertn.,
Digitaria violascens Link,
Amaranthus lividus Loisel.,
Polygonum blumei Meisn.,
Chenopodium album L.,
Chenopodium ficifolium Smith,
Amaranthus retroflexus L.,
Poa annua L.,
Rorippa palustris Bess.,
Polygonum nepalense Meisn.,
Rumex obtusifolius L.,
Rumex japonicus Houtt.,
Lamium amplexicaule L.,
Galium spurium L.,
Stellaria alsine Grimm.,
Cardamine flexuosa With. und
Polygonum ariculare L.

Die Verbindungen gemäss der vorliegenden Erfindung können sicher und ohne Schadwirkung bei vielen Nutzpflanzen wie Bohnen, Weizen, Baumwolle, Möhren, Kartoffeln, Rüben, Kohl, Senf, Erdnüssen, Rettich, Tabak, Tomaten und Gurken angewandt werden.

Die Einsatzmöglichkeiten der aktiven Verbindungen gemäss der vorliegenden Erfindung sind nicht auf Unkräuter auf höher gelegenen Anbauflächen beschränkt, sondern die Verbindungen sind ebenso auch gegen Schadunkräuter bei Reis, bei Binsen und auf Brachflächen wirksam. Der Begriff «Unkräuter», wie er hierin verwendet wird, bezeichnet im weitesten Sinne alle Pflanzen, die an Orten wachsen, an denen sie unerwünscht sind.

Die Verbindungen der allgemeinen Formel (I) gemäss der vorliegenden Erfindung können beispielsweise mittels des folgenden Verfahrens i) hergestellt werden.

*Verfahren i)*

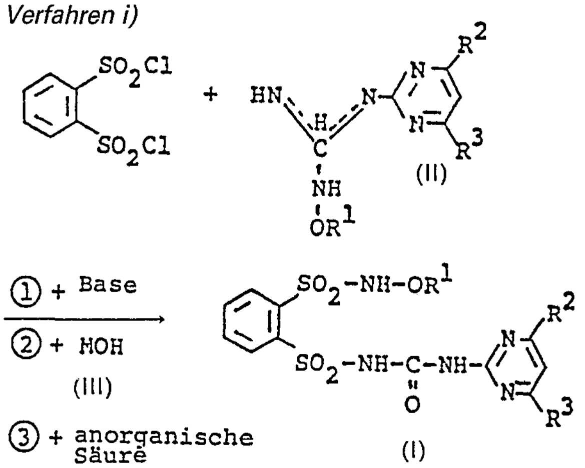


(II)

(I)

(In den Formeln haben $R^1$, $R^2$, $R^3$ und M die im Vorstehenden angegebenen Bedeutungen.)

In dem vorstehenden Reaktionsschema bezeichnen

$R^1$, $R^2$ und $R^3$ jeweils eine niedere Alkyl-Gruppe mit 1 bis 4 Kohlenstoff-Atomen wie Methyl, Ethyl, Propyl, Isopropyl und n- (iso, sec- und tert-) -Butyl.

M steht für ein Alkalimetall-Atom wie Lithium, Natrium und Kalium.

Zu speziellen Beispielen für die Verbindungen der allgemeinen Formel (II) als Ausgangsstoffe in dem vorstehenden Reaktionsschema zählen

N-(4,6-Dimethylpyrimidin-2-yl)-N'-methoxyguanidin,

N-(4,6-Dimethylpyrimidin-2-yl)-N'-ethoxyguanidin.

Zu speziellen Beispielen für die Alkalimetallhydroxide der allgemeinen Formel (III) zählen Natriumhydroxid, Kaliumhydroxid und Lithiumhydroxid.

Spezielle Beispiele für die anorganische Säure sind Salzsäure und Schwefelsäure.

Pyridin ist als spezielles Beispiel für die Base zu nennen.

Das vorstehende Verfahren wird anhand des folgenden typischen Beispiels speziell beschrieben.

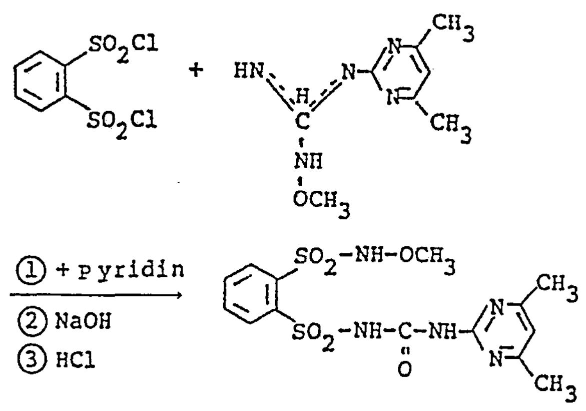


Zweckmässigerweise kann das vorstehende Verfahren zur Herstellung der Verbindungen der vorliegenden Erfindung unter Verwendung eines Lösungsmittels oder Verdünnungsmittels durchgeführt werden. Zu diesem Zweck können sämtliche inerten Lösungsmittel und Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die obige Reaktion kann in Gegenwart eines säurebindenden Mittels durchgeführt werden. Beispiele für das säurebindende Mittel umfassen die Hydroxide, Carbonate, Hydrogencarbonate und Alkoholate von Alkalimetallen, sowie tertiäre Amine wie Triethylamin, Diethylanilin und Pyridin, die sämtlich allgemein verwendet werden.

Das Verfahren gemäss der vorliegenden Erfindung kann in einem weiten Temperaturbereich durchgeführt werden. Im allgemeinen kann es bei einer Temperatur zwischen etwa −20° C und dem Siedepunkt der Mischung, zweckmässigerweise zwischen etwa 0° C und etwa 100° C, durchgeführt werden. Die Reaktion wird zweckmässigerweise unter normalem Atmosphärendruck durchgeführt, jedoch ist es auch möglich, unter erhöhtem oder vermindertem Druck zu arbeiten.

Als Herbizide können die Verbindungen der allgemeinen Formel (I) gemäss der vorliegenden Erfindung als solche unmittelbar nach dem Verdünnen mit Wasser oder in Form verschiedenartiger Präparate, wie sie gewöhnlich mittels der in der Herstellung von Agrochemikalien üblichen Verfahren unter Verwendung von landwirtschaftlich unbedenklichen Hilfsstoffen erhalten werden, angewandt werden. Beim praktischen Einsatz werden diese verschiedenartigen Präparate entweder unmittelbar oder nach dem Verdünnen mit Wasser auf die gewünschten Konzentrationen ausgebracht.

Beispiele für die agrochemisch unbedenklichen Hilfsstoffe, auf die hier Bezug genommen wird, umfassen beispielsweise Verdünnungsmittel (Lösungsmittel, Streckmittel, Träger), oberflächenaktive Stoffe (Lösungsvermittler, Emulgatoren, Dispergiermittel, Netzmittel), Stabilisatoren, Haftmittel, Aerosol-Treibmittel und Synergisten.

Beispiele für die Lösungsmittel sind Wasser und organische Lösungsmittel, beispielsweise Kohlenwasserstoffe [z.B. n-Hexan, Petrolether, Erdöl-Fraktionen (z.B. Paraffinwachse, Kerosin, Leichtöle, Mittelöle und Schweröle), Benzol, Toluol und Xylol], halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methylalkohol, Ethylalkohol, Propylalkohol und Ethylenglycol), Ether (z.B. Diethylether, Ethylenoxid und Dioxan), Alkoholether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Schwefel, Löschkalk, Magnesiumkalk, Gips, Calciumcarbonat, Silicium-dioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylsulfonsäureester (z.B. Natriumlaurylsulfat), Arylsulfonsäure-Salze (z.B. Alkylarylsulfonsäure-Salze und Natriumalkylnaphthalinsulfonate), Bernsteinsäure-Salze und Salze von Schwefelsäureestern von Polyethylenglycol-alkyl-arylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas (LNG) sowie niedere Ether), die Verbrennung steuernde Mittel für Räuchermittel (z.B. Nitrite, Zink-Pulver und Dicyandiamid), Sauerstoff abgebende Mittel (z.B. Chlorate), wirkungsverlängernde Mittel, Dispersions-Stabilisatoren (z.B. Casein, Tragant, Carboxymethylcellulose (CMC) und Polyvinylalkohol (PVA)) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate, benetzbare Pulver, lösliche Pulver, Suspensionen, Stäubemittel, Granulat und pulvrige Präparate.

Die Herbizide gemäss der vorliegenden Erfindung können von etwa 0,001 bis etwa 100 Gew.-%, vorzugsweise etwa von 0,005 bis etwa 95 Gew.-%, der vorerwähnten Wirkstoffe enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Art des Präparats, dem Verfahren, Zweck, der Zeit und dem Ort seiner Anwendung, dem Zustand des Auftretens der Unkräuter etc. variiert werden.

Erforderlichenfalls können die Verbindungen der allgemeinen Formel (I) gemäss der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffen [z.B. Organophosphat-Verbindungen, Carbamat-Verbindungen, Dithio(oder thiol)-carbamat-Verbindungen, organischen Chlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen] und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Ausbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Giessen etc.) und Bodenbehandlung (Vermischen mit dem Boden, Streuen etc.). Sie können auch mittels des sogenannten Ultra-Low-Volume-Sprühverfahrens ausgebracht werden. Nach diesem Verfahren kann der Wirkstoff sogar in einer Konzentration von 100% zur Anwendung gelangen.

Die Aufwandmenge pro Flächeneinheit beträgt beispielsweise etwa 0,005 bis etwa 3 kg/ha, vorzugsweise etwa 0,01 bis etwa 1 kg/ha. In speziellen Fällen können jedoch oder sollten sogar die Aufwandmengen ausserhalb des angegebenen Bereichs liegen.

Gemäss der vorliegenden Erfindung kann ein herbizides Mittel zur Verfügung gestellt werden, das als Wirkstoff eine Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel oder ein synergistisches Mittel enthält.

Die vorliegende Erfindung macht weiterhin ein Verfahren zur Unkrautbekämpfung verfügbar, das darin besteht, dass eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel oder einem synergistischen Mittel auf Unkräuter und/oder ihren Lebensraum ausgebracht wird.

Die vorliegende Erfindung wird durch die folgenden Beispiele näher erläutert, ist jedoch nicht auf diese speziellen Beispiele allein beschränkt.

*Beispiel 1:*

N-(4,6-Dimethylpyrimidin-2-yl)-N'-methoxyguanidin (1,95 g) wurde in Pyridin (50 ml) gelöst, und 1,2-Benzolsulfonylchlorid (2,75 g) wurde hinzugegeben. Die Mischung wurde einen Tage bei Raumtemperatur gerührt. Nach der Reaktion wurde das Pyridin unter vermindertem Druck aus der Reaktionsmischung abgedampft. Eine wässerige 2N Natriumhydroxid-Lösung (50 ml) wurde dem Rückstand zugesetzt, und die Mischung wurde 1 h bei Raumtemperatur gerührt. Beim Einstellen der wässerig-alkalischen Lösung mit Salzsäure auf pH 1 fielen rohe Kristalle aus. Die Kristalle wurden durch Filtration gesammelt und aus Acetonitril umkristallisiert, wonach der gewünschte N-(2-Methoxyaminosulfonylbenzolsulfonyl)-N'-(4,6-dimethylpyrimidin-2-yl)harnstoff (2,5 g) der nachstehenden Formel erhalten wurde.

Schmp. 218-219° C.

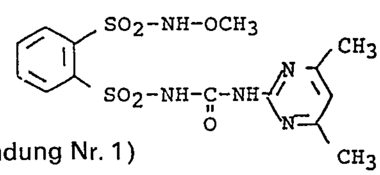

(Verbindung Nr. 1)

Die Struktur der vorstehenden Verbindung wurde mittels Röntgenbeugungsanalyse analysiert und bestimmt.

Mittels analoger Arbeitsweise wie in Beispiel 1 wurde N-(2-Ethoxyaminosulfonylbenzolsulfonyl)-N'-(4,6-dimethylpyrimidin-2-yl)harnstoff der nachstehenden Formel synthetisiert.

Schmp. 221-222° C.

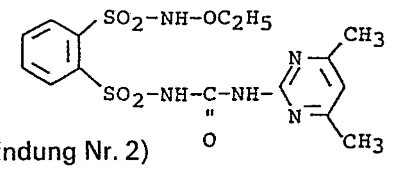

(Verbindung Nr. 2)

Die in der folgenden Tabelle 1 aufgeführten Verbindungen wurden nach analoger Arbeitsweise wie in Beispiel 1 unter Einsatz passend ausgewählter Guanidine der allgemeinen Formel (II), Metallhydroxide der allgemeinen Formel (III) und anorganischer Säuren synthetisiert.

*Tabelle 1*

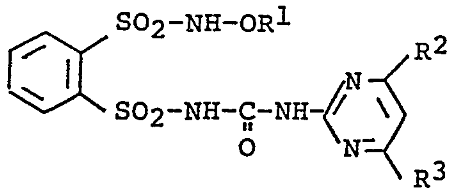

(I)

| Verbindung Nr. | $R^1$ | $R^2$ | $R^3$ | Physikal. Konstante |
|---|---|---|---|---|
| 3 | $-CH_3$ | $-C_2H_5$ | $-C_2H_5$ | |
| 4 | $-CH_3$ | $-C_3H_7$-n | $-C_3H_7$-n | |
| 5 | $-CH_3$ | $-C_3H_7$-iso | $-C_3H_7$-iso | |
| 6 | $-C_2H_5$ | $-C_2H_5$ | $-C_2H_5$ | |
| 7 | $-C_3H_7$-n | $-CH_3$ | $-CH_3$ | Fp. 212° C (Zers.) |
| 8 | $-C_4H_9$-n | $-CH_3$ | $-CH_3$ | Fp. 169° C |
| 9 | $-C_3H_7$-iso | $-CH_3$ | $-CH_3$ | Fp. 218° C (Zers.) |
| 10 | $-CH_3$ | $-C_4H_9$-n | $-C_4H_9$-n | |

*Beispiel 2:*

Benetzbares Pulver.

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgetropft.

*Beispiel 3:*

Emulgierbares Konzentrat.

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgetropft.

*Beispiel 4:*

Stäubemittel.

2 Teile der Verbindung Nr. 1 der Erfindung und 98 Teile Tonpulver wurden pulverisiert und gemischt, wodurch ein Stäubemittel hergestellt wird. Dieses wird über Unkräutern und/oder ihrem Lebensraum ausgestreut.

*Beispiel 5:*

Stäubemittel.

Die Verbindung Nr. 2 der Erfindung (1,5 Teile), 0,5 Teile Isopropylhydrogenphosphat (PAP) und 98 Teile Tonpulver wurden pulverisiert und gemischt, wodurch ein Stäubemittel hergestellt wird. Dieses wird über Unkräutern und/oder ihrem Lebensraum ausgestreut.

*Beispiel 6:*

Granulat.

Wasser (25 Teile) wird zu einer Mischung aus 10 Teilen der Verbindung Nr. 1 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngrösse von 0,43 bis 2,0 mm (10 bis 40 mesh) verarbeitet und bei 40° C bis 50° C getrocknet, wodurch ein Granulat gebildet wird. Dieses wird über Unkräutern und/oder ihrem Lebensraum ausgestreut.

*Beispiel 7:*

Granulat.

Ein Drehmischer wird mit 95 Teilen Tonmineral-Teilchen mit einer Teilchengrössen-Verteilung im Bereich von 0,2 bis 2 mm beschickt, und unter Drehen des Mischers werden 5 Teile der Verbindung Nr. 15 der Erfindung, gelöst in einem organischen Lösungsmittel, zur gleichmässigen Benetzung auf die Teilchen aufgesprüht, und die Teilchen werden bei 40° C bis 50° C getrocknet, wodurch ein Granulat gebildet wird. Dieses wird über Unkräutern und/oder ihrem Lebensraum ausgestreut.

*Beispiel 8 (Biologischer Test)*

Prüfung der Wirkung gegen Acker-Unkräuter und Nutzpflanzen durch Behandlung des Laubs nach dem Auflaufen:

*Wirkstoff-Präparat:*

Träger: 5 Gewichtsteile Aceton;
Emulgator: 1 Gewichtsteil Benzyloxypolyglycolether.

Zur Herstellung eines Wirkstoff-Präparats wurde 1 Gew.-Teil jeder der aktiven Verbindungen mit dem Träger und Emulgator in den oben bezeichneten Mengen unter Bildung eines emulgierbaren Konzentrats vermischt, und dieses wurde mit Wasser auf eine vorher festgelegte Konzentration verdünnt.

*Test-Verfahren:*

In einem Gewächshaus wurde Weizen-Satgut in mit Ackerboden gefüllte Töpfe (10 $dm^2$) eingesät, und Erde, die Samen jeder der Species Alopecurus aequalis Sobol. var. amurensis Ohwi, Stellaria media Villars. und Stellaria alsine Grim. enthielt, wurde auf den Boden aufgebracht, so dass sie diesen bis zu einer Tiefe von 1 cm bedeckte.

10 Tage nach dem Keimen (wenn der Weizen und die Unkrautarten sich im Zwei-Blatt-Stadium befanden), wurde jede der wie oben mit einer bestimmten Konzentration zubereiteten Chemikalien gleichmässig auf die oberflächenschicht des Bodens in jedem der Test-Töpfe aufgesprüht.

Vier Wochen nach dem Sprühen wurden die herbizide Wirkung und der Grad der Phytotoxizität gegenüber Weizen mit Hilfe einer Skala von 0 bis 5 nach dem folgenden Massstab bewertet.

Bewertung der herbiziden Wirkung
(Herbiziditätsrate,
bezogen auf die unbehandelte Fläche)

| | |
|---|---|
| 5 | wenigstens 95% (verdorrt) |
| 4 | wenigstens 80%, jedoch weniger als 95% |
| 3 | wenigstens 50%, jedoch weniger als 80% |
| 2 | wenigstens 30%, jedoch weniger als 50% |
| 1 | wenigstens 10%, jedoch weniger als 30% |
| 0 | weniger als 10% (unwirksam) |

Bewertung der Phytotoxizität gegenüber Weizen
(Phytotoxizitätsrate,
bezogen auf die unbehandelte Fläche)

| | |
|---|---|
| 5 | wenigstens 90% (Ausrottung) |
| 4 | wenigstens 50%, jedoch weniger als 90% |
| 3 | wenigstens 30%, jedoch weniger als 50% |
| 2 | wenigstens 10%, jedoch weniger als 30% |
| 1 | mehr als 0%, jedoch weniger als 10% |
| 0 | 0% (keine Phytotoxizität) |

Ein Phytotoxizitäts-Index von 2 oder mehr bedeutet, dass das Mittel nicht praktisch einsetzbar ist.

Die Ergebnisse sind in Tabelle 2 aufgeführt.

*Tabelle 2*

| Verbindung Nr. | Wirkstoff-Menge kg/ha | Herbizide Wirkung A. aequalis | Herbizide Wirkung S. media | Herbizide Wirkung S. alsine | Phyto-toxizität gegen Weizen |
|---|---|---|---|---|---|
| 1 | 0,05 | 5 | 5 | 5 | 0 |
| | 0,01 | 4 | 5 | 5 | 0 |
| 2 | 0,05 | 5 | 5 | 5 | 0 |
| | 0,01 | 4 | 5 | 5 | 0 |
| Kontrolle: | | | | | |
| Z-1 | 0,05 | 5 | 5 | 5 | 2 |
| | 0,01 | 2 | 4 | 3 | 0 |
| Simazine | 0,4 | 3 | 5 | 5 | 1 |

*Anmerkung:*

1. Simazin (Handelsprodukt): 2-Chloro-4,6-bis(ethyl-amino)-1,3,5-triazin (50-proz. benetzbares Pulver).
2. Z-1:

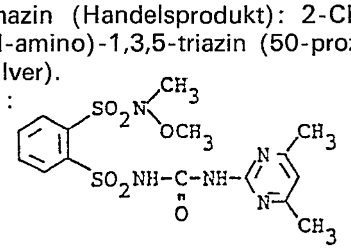

(die in der EP-A-23141 beschriebene Verbindung).

*Beispiel 9 (Biologischer Test)*

Prüfung der Wirkung gegen Acker-Unkräuter und Nutzpflanzen durch Vorauflauf-Behandlung des Bodens:

Test-Verfahren:

In einem Gewächshaus wurde Sojabohnen-Saatgut in mit Ackerboden gefüllte Töpfe (10 dm²) eingesät, und Erde, die Samen jeder der Species Digitaria adscendens Henr., Amaranthus lividus Loisel., Chenopodium album L. und Echinochloa crus-galli P. Beauv. enthielt, wurde auf den Boden aufgebracht, so dass sie diesen bis zu einer Tiefe von 1 cm bedeckte. Einen Tag nach dem Säen und Abdecken mit Erde wurde die hergestellte Chemikalie mit einer vorher festgelegten Konzentration gleichmässig auf die Oberflächenschicht des Bodens in jedem der Test-Töpfe aufgesprüht.

Vier Wochen nach dem Sprühen wurden die herbizide Wirkung und der Grad der Phytotoxizität nach dem gleichen Massstab wie in Beispiel 8 bewertet.

Die Ergebnisse sind in Tabelle 3 aufgeführt.

*Tabelle 3*

| Verbindung Nr. | Wirkstoff-Menge kg/ha | Herbizide Wirkung D. adscendens | A. lividus | C. album | E. crus-galli | Phyto-toxizität gegen Sojabohnen |
|---|---|---|---|---|---|---|
| 1 | 0,1 | 5 | 5 | 5 | 5 | 0 |
| 2 | 0,1 | 5 | 5 | 5 | 5 | 0 |
| Kontrolle: | | | | | | |
| Z-1 | 0,1 | 5 | 5 | 5 | 5 | 3 |
| Simazine | 0,4 | 3 | 3 | 5 | 5 | 1 |

*Anmerkung:*

Z-1 und Simazin sind die gleichen Verbindungen, wie in der Fussnote zu Tabelle 2 angegeben.

## Patentansprüche

1. 2-Alkoxyaminosulfonylbenzolsulfonyl-harnstoff-Derivate der allgemeinen Formel (I)

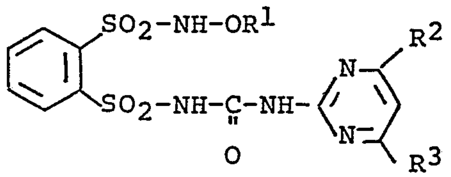

(I)

in der R¹, R² und R³ jeweils eine Alkyl-Gruppe mit 1 bis 4 Kohlenstoff-Atomen bezeichnen.

2. N-(2-Methoxyaminosulfonylbenzolsulfonyl)-N'-(4,6-dimethylpyrimidin-2-yl)harnstoff nach Anspruch 1, gekennzeichnet durch die folgende Formel

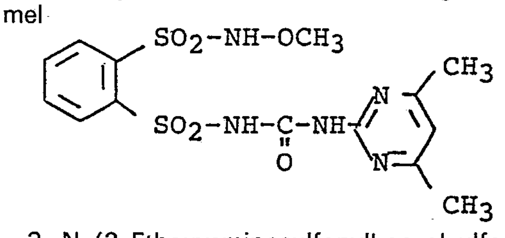

3. N-(2-Ethoxyaminosulfonylbenzolsulfonyl)-N'-(4,6-dimethylpyrimidin-2-yl)harnstoff nach Anspruch 1, gekennzeichnet durch die folgende Formel

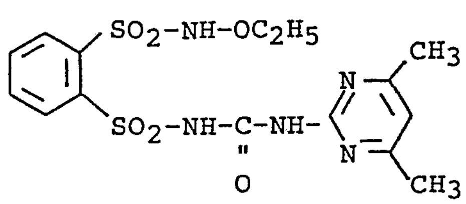

4. Verfahren zur Herstellung von 2-Alkoxyaminosulfonylbenzolsulfonyl-harnstoff-Derivaten der allgemeinen Formel (I)

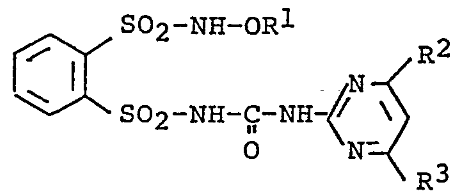

(I)

in der R¹, R² und R³ jeweils eine Alkyl-Gruppe mit 1 bis 4 Kohlenstoff-Atomen bezeichnen, dadurch gekennzeichnet, dass eine Verbindung der Formel

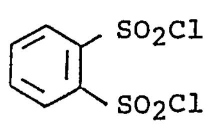

mit einer Verbindung der allgemeinen Formel

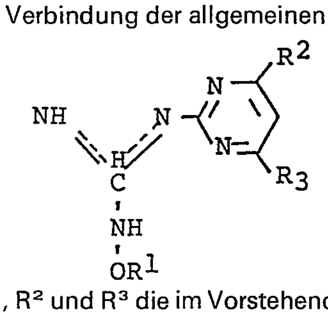

(II)

in der R¹, R² und R³ die im Vorstehenden angegebene Bedeutung haben,
in Gegenwart einer Base umgesetzt wird, das Produkt anschliessend mit einem Alkalimetallhydroxid der allgemeinen Formel

M-OH (III)

in der M ein Alkalimetall-Atom bezeichnet, umgesetzt wird und weiterhin das Produkt mit einer anorganischen Säure umgesetzt wird.

5. Herbizide Mittel, gekennzeichnet durch einen Gehalt an mindestens einem 2-Alkoxyaminosulfonylbenzolsulfonylharnstoff-Derivat der allgemeinen Formel (I) gemäss Anspruch 1.

6. Verwendung von 2-Alkoxyaminosulfonylbenzolsulfonylharnstoff-Derivaten der allgemeinen Formel (I) gemäss Anspruch 1 zur Bekämpfung von unerwünschtem Pflanzenwachstum.

7. Verfahren zur Herstellung von herbiziden Mitteln, dadurch gekennzeichnet, dass man 2-Alkoxyaminosulfonylbenzolsulfonyl-harnstoff-Derivate der allgemeinen Formel (I) gemäss Anspruch 1 mit Streckmitteln und/oder oberflächenaktiven Mitteln vermischt.

## Claims

1. 2-Alkoxyaminosulphonylbenzenesulphonylurea derivatives of the general formula (I)

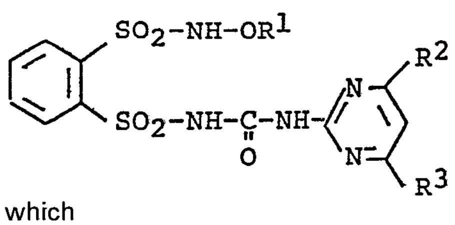

(I)

in which

$R^1$, $R^2$ and $R^3$ each designate an alkyl group with 1 to 4 carbon atoms.

2. N-(2-methoxyaminosulphonylbenzenesulphonyl)-N'-(4,6-dimethylpyrimidin-2-yl)urea according to Claim 1, characterised by the following formula

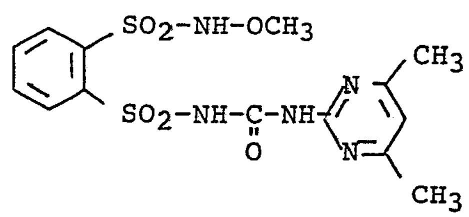

3. N-(2-ethoxyaminosulphonylbenzenesulphonyl)-N'-(4,6-dimethylpyrimidin-1-yl)urea according to Claim 1, characterised by the following formula

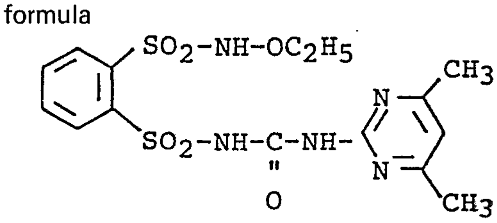

4. Process for the preparation of 2-alkoxyaminosulphonylbenzenesulphonylurea derivatives of the general formula (I)

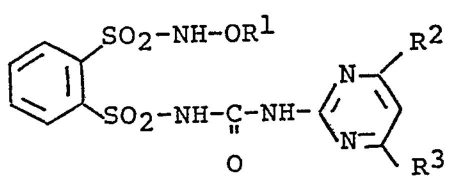

(I)

in which

$R^1$, $R^2$ and $R^3$ each designate an alkyl group with 1 to 4 carbon atoms, characterised in that a compound of the formula

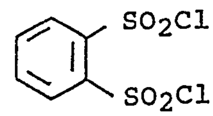

is reacted with a compound of the general formula

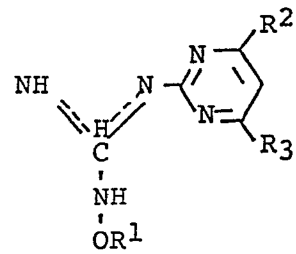

(II)

in which

$R^1$, $R^2$ and $R^3$ have the meaning given above, in the presence of a base, the product is then reacted with an alkali metal hydroxide of the general formula

$$M-OH \qquad (III)$$

in which

M designates an alkali metal atom, and the product is further reacted with an inorganic acid.

5. Herbicidal agents, characterised in that they contain at least one 2-alkoxyaminosulphonylbenzenesulphonylurea derivative of the general formula (I) according to Claim 1.

6. Use of 2-alkoxyaminosulphonylbenzenesulphonylurea derivatives of the general formula (I) according to Claim 1 for combating undesired plant growth.

7. Process for the preparation of herbicidal agents, characterised in that 2-alkoxyaminosulphonylbenzenesulphonylurea derivatives of the general formula (I) according to Claim 1 are mixed with extenders and/or surface-active agents.

## Revendications

1. Dérivés de 2-alcoxyaminosulfonylbenzènesulfonyl-urée de formule générale (I)

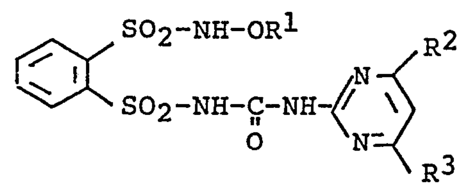

(I)

dans laquelle $R^1$, $R^2$ et $R^3$ désignent chacun un groupe alcoyle ayant 1 à 4 atomes de carbone.

2. N-(2-méthoxyaminosulfonylbenzènesulfonyl)-N'-(4,6-diméthylpyrimidine-2-yl)urée selon la revendication 1, caractérisée par la formule suivante:

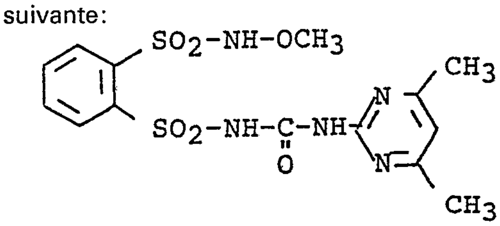

3. N-(2-éthoxyaminosulfonylbenzènesulfonyl)-N'-(4,6-diméthylpyrimidine-2-yl)urée selon la revendication 1, caractérisée par la formule suivante:

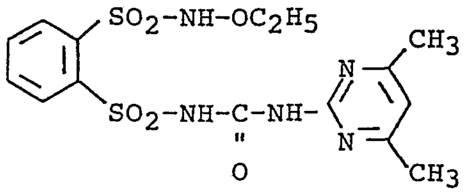

4. Procédé de fabrication de dérivés de 2-alcoxyaminosulfonylbenzènesulfonyl-urée de formule générale (I)

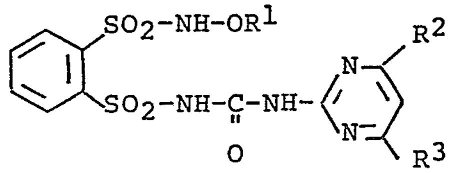

(I)

dans laquelle $R^1$, $R^2$ et $R^3$ signifient chacun un groupe alcoyle ayant 1 à 4 atomes de carbone, caractérisé en ce qu'on fait réagir un composé de formule:

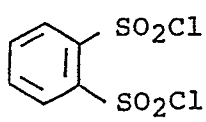

avec un composé de formule générale:

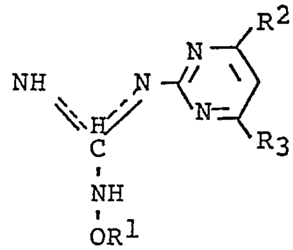

(II)

dans laquelle $R^1$, $R^2$ et $R^3$ ont la signification indiquée plus haut, en présence d'une base, en ce qu'on fait ensuite réagir le produit avec un hydroxyde de métal alcalin de formule générale:

$$M-OH \qquad (III)$$

et en ce qu'on fait en outre réagir le produit avec un acide minéral.

5. Agents herbicides, caractérisés par une teneur en au moins un dérivé de 2-alcoxyaminosulfonylbenzènesulfonyl-urée de formule générale (I) selon la revendication 1.

6. Utilisation de dérivés de 2-alcoxyaminosulfonylbenzènesulfonyl-urée de formule générale (I) selon la revendication 1 pour combattre la croissance indésirable des plantes.

7. Procédé de fabrication d'agents herbicides, caractérisé en ce qu'on mélange des dérivés de 2-alcoxyaminosulfonylbenzènesulfonyl-urée de formule générale (I) selon la revendication 1 avec des diluants et/ou des agents tensio-actifs.